# EUROPEAN PATENT APPLICATION

(11) **EP 4 230 201 A1**
(43) Date of publication of application: **23.08.2023**
(21) Application number: 22166594.6
(22) Date of filing: 04.04.2022
(51) Int. Cl.: A61K 31/255, A61K 31/191, A61K 31/192, A61K 31/216, A61K 31/235, A61P 25/08, A61P 25/14, A61P 25/16, A61P 25/28

(54) **COMPOSITION FOR TREATING NEURODEGENERATIVE DISEASES**

(30) Priority: 21.02.2022 PT 2022117801
(71) Applicant: UNIVERSIDADE NOVA DE LISBOA, 1099-085 Lisboa (PT)
(72) Inventor: GODINHO FERREIRA DIAS NUNES DOS SANTOS, MARIA C., 1150-082 LISBOA (PT); CARREGOSA, DIOGO MIGUEL JOSÉ, 1150-082 LISBOA (PT); MERCA SARAIVA MONTEIRO CARECHO, RAFAEL JOSÉ, 1150-082 LISBOA (PT)
(74) Representative: De Tullio, Michele Elio

(57) **Abstract**

Through the past decades, several epidemiological studies have revealed that polyphenols-rich Mediterranean diet, comprising fruit and vegetables, can provide beneficial effects in humans, preventing neurodegenerative diseases and cognitive decline. After ingestion, polyphenols undergo extensive metabolization, generating a wide range of metabolites, identified as the effective bioactive molecules in human organism. Provided herein is a polyphenol metabolitebased composition for preventing and treating neurodegenerative diseases. Proof-of-principle for the latter concept is demonstrated by *in vitro* reduction in multiple cytokines production, usually associated to neuroinflammation and neurodegenerative diseases. The composition of the present invention can be used in nutraceutical and pharmaceutical formulations.

## Description

### TECHNICAL FIELD

The present invention relates to a polyphenol metabolites-based composition. More particularly, the present invention relates to polyphenol metabolites-based composition for preventing and/or treating neuroinflammation and/or neurodegenerative diseases.

### BACKGROUND AND STATE OF THE ART

It has been comprehensively understood that with increased life expectancy, the world's population is getting older with concomitant incidence and over time acceleration of age-related diseases such as neurodegenerative diseases, nervous system dysfunction and syndromes ultimately leading to impaired mental and movement function. A huge number of neurodegenerative diseases, comprising Alzheimer's disease, Parkinson's diseases, amyotrophic lateral sclerosis, muscular dystrophy, multiple sclerosis, brain cancer, epilepsy, are known to affect the nervous system. The determinant role of several factors like misfolded protein aggregation, increased oxidative stress, lipid peroxidation, DNA damage, uncontrolled signalling and neuroinflammation, have begun to emerge as a relevant focus to develop new interventions tackling disease progression. Current therapies only alleviate physical complications, being unable to abolish the pathology of this burden to society. These factors make the discovery of novel therapies to delay and prevent the development of these diseases an urgent yet unmet need.

Neuroinflammation appears as one of the key processes involved in major neurodegenerative diseases, being not only essential for recovery from several conditions but also playing detrimental roles contributing to disease acceleration. This process is controlled by microglia cells, the innate immune cells of the central nervous system (CNS). It is commonly acknowledged that microglia play a major role in neurological and neurodegenerative diseases (see Prokop et al., Acta Neuropathol 126 (2013), 461-77 for review), engaging a first line of defence against invading pathogens or other types of brain tissue injury and are understood to be the "sentinels" of CNS. Under transient damage microglia cells activate to protect their surrounding microenvironment and maintain the normal functions of CNS playing immune resolving, ant-inflammatory functions and supporting cell renewal by secreting trophic factors. Vice versa, under intensive pathological acute or chronic activation, usually associated with neurodegenerative disease, stroke and tumour invasion, microglia cells become neurotoxic and secrete a plethora of pro-inflammatory cytokines, such us for example TNF-α, IL-6, IL-10, IL-1β or prostaglandins synthetized by COX-2, and reactive oxygen (ROS) and nitrogen (NOS) species, that may potentially impair neuronal activity, leading to disturbance of normal neurotransmitter function and irreversible tissue loss. The main signalling cascade behind the activation of microglia cells involves Nuclear Factor kappa B (NF-kB) through a mitogen-activated protein kinase (MAPK) signal transduction pathway. Additionally, transmembrane protein toll-like receptor 4 (TLR4) is also involved in activation of NF-kB and production of pro-inflammatory cytokines. NF-kB transcription factor is one of the most important molecular mediators in inflammation, also involved in tumour promoting processes (Karin M, Greten FR. NF-kB: linking inflammation and immunity to cancer development and progression. Nat Rev Immunol. 2005 Oct; 5(10):749-59).

Since NF-kB identification and its inactivation due to regulatory protein IkB-α, it has been suggested that many of the proteins involved in its activation pathway, and hence responsible for inflammation and cancer, can be molecular targets for many drugs. Some of these drugs have been discovered so far, and some are being tested in clinical essays (Karin M, Yamamoto Y, Wang QM. The IKK NF-kB system: a treasure trove for drug development. Nat Rev Drug Discov. 2004 Jan; 3(1):17-26). Accordingly, targeting microglia cells provides an interesting new avenue in the development of novel treatments for these diseases. Current approaches mostly aim at reducing microglia activation and production of pro-inflammatory cytokines in order to avoid neuroinflammation-mediated neuronal damage (reviewed, e.g., in Solito and Sastre, Front Pharmacol 3 (2012), and Wang et al., Ann. Transl. Med. 3 (2015): 136). Genetic variations in the triggering receptor expressed on myeloid cells 2 (TREM2) reported to disturb proper microglia function (loss-of-function variants) are linked to a spectrum of neurodegenerative disorders including Alzheimer's disease (AD), frontotemporal dementia (FTD), Parkinson's disease (PD), amyotrophic lateral sclerosis (ALS) and Nasu-Hakola disease (NHD, also known as polycystic lipomembranous osteodysplasia with sclerosing leukoencephalopathy, PLOSL) (reviewed in Yeh et al., Trends Mol. Med. 23 (2017), 512-533). TREM2 is expressed by microglia and promotes microglia survival, activation, and the formation of a protective microglia barrier around amyloid plaque. Studies have shown that TREM2-mediated activities are intimately involved in neurodegenerative diseases and therefore provide a new therapeutic target. Specifically, the in vitro and in vivo treatment of microglia and mouse hippocampi with transgenic soluble TREM2 (sTREM2) protein revealed a protective effect on microglial viability and a trigger of microglial activation (Zhong et al., J. Exp. Med. 214 (2017), 597-607). However, the application of a genetically engineered protein-based drugs as shown in KR 2019-7029338 A, JP 2017-74982 A and WO20140330704A1 is highly inconvenient, cumbersome and expensive as well as at risk of eliciting an undesired immunogenic response to the protein therapeutic and inadequate exposure due to poor passage through blood-brain-barrier (BBB), the dynamic interface limiting molecular exchanges between the blood and the neuronal tissue and having a crucial role in controlling the accessibility of nutrients and drugs to the brain.

Accordingly, there is still a need for therapies that promote microglia survival and activation for a safe and tolerable treatment of neurological and neurodegenerative diseases, capable of overcoming the drawbacks above mentioned.

These objectives are achieved with the present invention in accordance with the embodiments as characterized in the claims and described further below. Through the past decades, several epidemiological studies have revealed that polyphenols-rich diets, comprising fruit and vegetables, can provide beneficial effects in humans, preventing neurodegenerative disorders and cognitive decline. In fact, adherence to Mediterranean diet, rich in polyphenols, is associated with a reduction of the incidence of neurodegenerative disorders. Olives and berries are amongst the most promising fruits as sources of polyphenols with these health benefits. It is well acknowledged that hydroxytyrosol, oleuropein aglycone and other molecules having a polyphenolic structure are potent antioxidant and anti-inflammatory agents. Oleuropein also reveals a coronary-dilating, hypoglycemic and anti-cholesterolemic activity and, similarly to hydroxytyrosol, it delays oxidation of LDLs (Low Density Lipoprotein). Hydroxytyrosol has been shown to reduce the gene expression of NOS and COX-2 cell lines, thus preventing the activation of the NF-kB transcription factor.

Despite the accumulating evidence of beneficial effects, the basic mechanism of polyphenols action is still to be elucidated. Both indirect actions through peripheral effects and direct actions inside the brain have been suggested as potential mechanism. Most *in vitro* mechanistic studies with polyphenols have used pure components and do not deal with their metabolism and bioavailability. In many cases, the single molecule is less active with respect to the mixture of the compounds, thus suggesting a synergic action among them. Absorbed forms are not the native dietary compounds, and they are more likely to be a plethora of circulating metabolites (such us for example sulfate, O-glucuronates, and/or methyl ether derivatives) resulting from extensive conjugation due to digestion, hepatic and colonic metabolism (Kroon P.A. et al, Am. J. Clin. Nutr. (2004), 80, 15-21). Additionally, the ability of dietary polyphenol compounds to directly influence the nervous system is related to the ability of their metabolites to cross BBB. In fact, evidence of BBB permeation from polyphenol metabolites, such as for example hydroxycinnamic, hydroxybenzenes, benzoic acid derivatives, is growing in the literature (Gasperotti M. et al, ACS Chem. Neurosci. (2015), 6, (8), 1341-1352).

Several documents are reported in the patent literature claiming nutraceutical and/or pharmaceutical compositions as antioxidants and neuroprotective agents, such us for example the teachings in WO2017160923A1, WO2013132376A1, WO199905391A1 and WO1999052524A1. Nevertheless, due to the use of non-psycotropic cannabinoid and/or flavonoid compounds and/or vegetable oil extracts, the bioavailability of crucial polyphenolic metabolites crossing BBB is low and the effects reported do not necessarily relate to what may occur *in vivo.* In addition, macrocyclic derivatives present in WO2013132376A1 can be expensive to prepare and purify.

The most important granted prior art reference is constituted by US9610256B2, disclosing methods of use for 2,5-dihydroxybenzene sulfonic acid for inhibiting cancer cell proliferation. Surprisingly, the concept of the present invention that polyphenolic metabolites could be directly used in nutraceutical and/or pharmaceutical compositions for preventing and treating neurodegenerative diseases has never been proposed. These objectives are achieved with the present invention.

Patents and other publications identified are expressly incorporated herein by reference for the purpose of describing and disclosing, for example, the methodologies described in such publications, prior to the filing date and that might be used in connection with the present invention.

### DISCLOSURE OF THE INVENTION

The inventive principle of the present invention is to use polyphenol metabolites compounds for preventing and/or treating neuroinflammation and/or neurodegenerative diseases.

Thus, the present invention refers to a composition for preventing and/or treating neuroinflammation and/or neurodegenerative diseases, comprising at least one polyphenol metabolite-based compound

Advantageously, the composition of the present invention comprises a very different approach to that currently used in the nutraceutical and/or pharmaceutical industry for preventing and/or treating neurodegenerative diseases. The composition of the present invention can greatly expand the number and nature of low cytotoxicity active compounds accessible to BBB and will therefore increase the diversity of treatments obtained, including but not limited to: Alzheimer's disease, Parkinson's diseases, amyotrophic lateral sclerosis, muscular dystrophy, multiple sclerosis, brain cancer, epilepsy.

### BRIEF DESCRIPTION OF DRAWINGS

Characteristics and advantages of the invention will become more apparent to those skilled in the Art from the following detailed description of a preferred embodiments of the invention, given by way of non-limiting example with reference to the annexed drawings, in which:

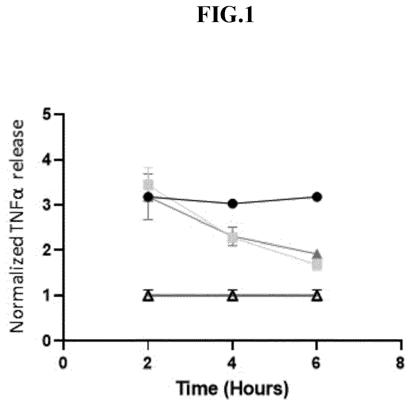

FIG. 1 shows the reduction in release of TNF-α for phloroglucinol-sulfate (3,5-dihydroxyphenyl hydrogen sulfate) and resorcinol-sulfate (3-hydroxyphenyl hydrogen sulfate) with 2, 4 and 6 hours of pre-incubation followed by 24 hours of incubation with lipopolysaccharides (black circles). Compounds were tested at a concentration of 100 nM. Light grey squares indicate resorcinol-sulfate, dark grey triangles indicate phloroglucinol-sulfate, white triangles indicate control conditions.

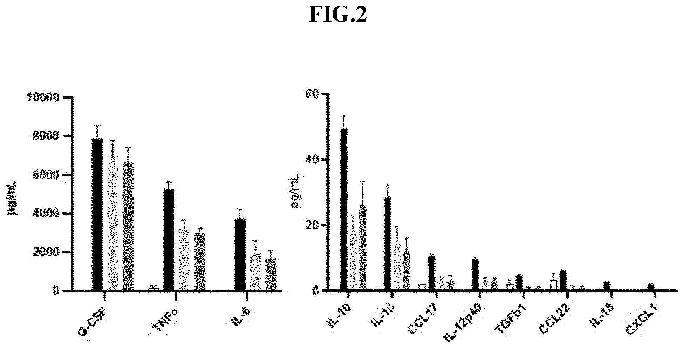

FIG. 2 shows the reduction in multiple cytokines comprising G-CSF, TNF-α, IL-6, IL-10, IL-1β, CCL17, IL-12p40, TGFβ1, CCL22, IL18, CXCL1 for phloroglucinol-sulfate and resorcinol-sulfate using a pre-incubation of 6 hours and compound concentration of 100 nM followed by 24 hours of inflammatory trigger with lipopolysaccharides (black bars). Light grey bars indicate resorcinol-sulfate, dark grey triangles indicate phloroglucinol-sulfate, white bars indicate control conditions.

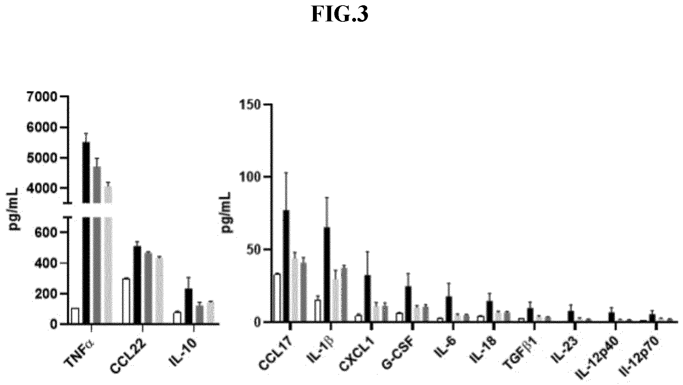

FIG. 3 shows the reduction in multiple cytokines comprising TNF-α, CCL22, IL-10, CCL17, IL-1β, CXCL1, G-CSF, IL-6, IL-18, TGFβ1, IL23, IL-12p40, IL-12p70 for phloroglucinol-sulfate and resorcinol-sulfate using a pre-incubation of 6 hours and compound concentration of 100 nM followed by 24 hours of inflammatory stimuli with TNF-α 10nM plus IFNγ 1nM. Light grey bars indicate resorcinol-sulfate, dark grey triangles indicate phloroglucinol-sulfate, white bars indicate control conditions.

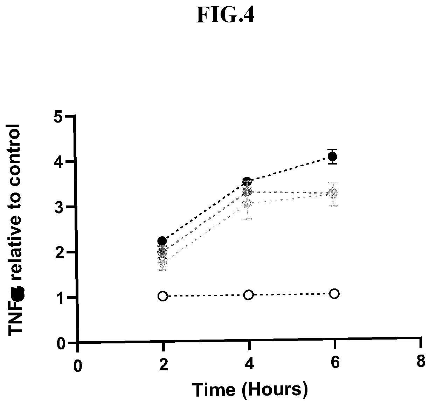

FIG. 4 shows the therapeutic model for phloroglucinol-sulfate and resorcinol-sulfate: cell were submitted to an inflammatory trigger with lipopolysaccharides (black circles) for 2 hours followed by compound incubation (100 nM) for 2, 4, 6 hours. Light grey circles indicate resorcinol-sulfate, dark grey circles indicate phloroglucinol-sulfate, white circles indicate control conditions.

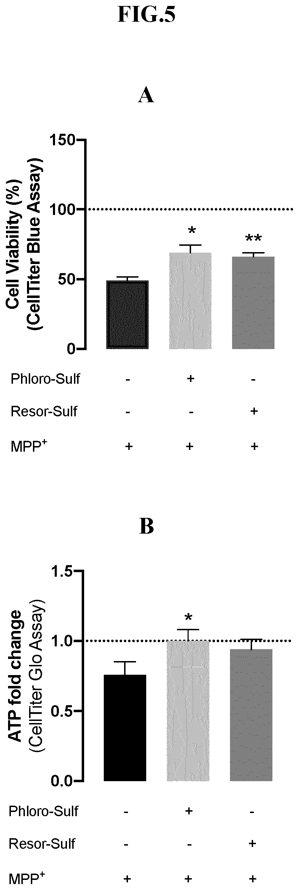

FIG. 5 shows the protective effects of 24 hours metabolites pre-treatment in (A) cell viability (metabolic capacity) and (B) ATP levels upon 24 hours of MPP+, mitigating the damage caused by an oxidative insult.

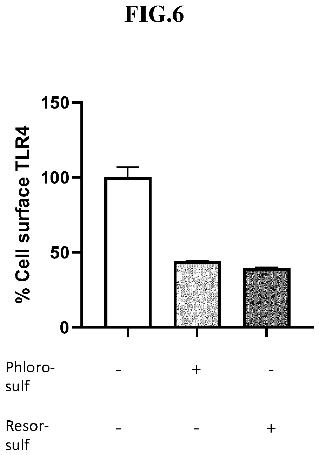

FIG. 6 shows the reduction of TLR4 in the outer cell membrane by 6 hours of compound incubation (100nM). White bar indicates control conditions, dark grey bar indicates phloroglucinol-sulfate, light grey bar indicates resorcinol-sulfate.

### DETAILED DESCRIPTION OF THE INVENTION

To facilitate understanding of the disclosure set forth herein, a number of terms are defined below.

Unless otherwise defined, scientific and technical terms used above and throughout the description, such as for example "neurodegenerative", "neurological", "polyphenol", "metabolite", "administration", "therapeutic" shall be intended to have the meanings that are commonly understood by those of ordinary skill in the Art to which this disclosure belongs.

In this specification and the appended claims, the singular forms "a," "an" and "the" include plural referents unless the context clearly dictates otherwise. The terms "a" (or "an"), as well as the terms "at least one" can be used interchangeably herein. In certain aspects, the term "a" or "an" means "single." In other aspects, the term "a" or "an" includes "two or more" or "multiple."

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone).

As used herein, the term "treating" means both therapeutic treatment and prophylactic or preventative measures wherein the object is to prevent or slow down an undesired physiological condition, disorder, or disease, or obtain beneficial or desired clinical results. Thus, those in need of treatment include those already diagnosed with or suspected of having the disorder. Beneficial or desired clinical results include, but are not limited to, alleviation of symptoms; diminishment of the extent of a condition, disorder, or disease; stabilized state of condition, disorder, or disease; delay in onset or slowing of condition, disorder, or disease progression; amelioration of the condition, disorder, or disease state or remission (whether partial or total), whether detectable or undetectable; an amelioration of at least one measurable physical parameter, not necessarily discernible by the patient; or enhancement or improvement of condition, disorder, or disease. Treatment includes eliciting a clinically significant response without excessive levels of side effects. Treatment also includes prolonging survival as compared to expected survival if not receiving treatment.

The term "therapeutically effective amount" is meant to include the amount of a compound that, when administered, is sufficient to prevent development of, or alleviate to some extent, one or more of the symptoms of a disorder, disease, or condition being treated. The term "therapeutically effective amount" also refers to the amount of a compound that is sufficient to elicit the biological or medical response of a cell, tissue, system, animal, or human, which is being sought by a researcher, veterinarian, medical doctor, or clinician.

The term "substantially pure" for agents, compounds, compositions, antibodies used in the methods described herein mean that said agents, compounds, compositions, antibodies are purchased and/or prepared separate from the components that normally accompany them.

As used herein, the term "nutraceutical" refers to a pharmaceutical-grade and standardized nutrient, usually food or parts of food that confers health benefits. They can be categorized as dietary supplements, pre- and probiotics, polyunsaturated fatty acids, antioxidants, and other types of herbal and natural foods. Popular nutraceuticals include ginseng, Echinacea, green tea, glucosamine, omega-3, lutein, folic acid, and cod liver oil. Because nutraceuticals are naturally occurring food parts, they confer fewer side effects and cost less than engineered pharmaceuticals.

The terms "pharmaceutically acceptable carrier", "pharmaceutically acceptable excipient", refer to a pharmaceutically acceptable material, composition, or vehicle, such as a liquid or solid excipient, solvent, or encapsulating material. In one aspect, each component is "pharmaceutically acceptable" in the sense of being compatible with the other ingredients of a pharmaceutical formulation, and suitable for use in contact with the tissue or organ of humans and animals without excessive toxicity, irritation, allergic response, immunogenicity, or other problems or complications, commensurate with a reasonable benefit/risk ratio. See Remington: The Science and Practice of Pharmacy, 21st Edition, Lippincott Williams & Wilkins: Philadelphia, Pa., 2005; Handbook of Pharmaceutical Excipients, 5th Edition, Rowe et al., Eds., The Pharmaceutical Press and the American Pharmaceutical Association: 2005; and Handbook of Pharmaceutical Additives, 3rd Edition, Ash and Ash Eds., Gower Publishing Company: 2007; Pharmaceutical Preformulation and Formulation, Gibson Ed., CRC Press LLC: Boca Raton, Fla., 2004. The term "pharmaceutical composition," as used herein, represents a composition containing a compound described herein formulated with a pharmaceutically acceptable excipient, and can be manufactured or sold with the approval of a governmental regulatory agency as part of a therapeutic regimen for the treatment of disease in a mammal.

Thus, in a first embodiment the present invention relates to a polyphenol metabolites-based composition comprising at least a therapeutically effective amount of one compound of Formula (I) 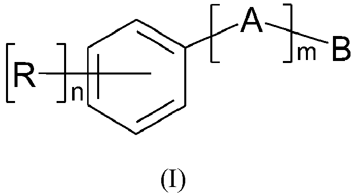 or salts thereof, wherein:
[R]n is one radical R if n is 1 or represents n radicals R attached to different position of the basic ring if n is more than 1, and wherein each R is independently selected from the group consisting of H, OH, OCH₃, OCH₂CH₃, OSO₃H, OSO₃CH₃, OSO₃CH₂CH₃, F, Cl, Br, I, preferably OH, OCH₃, OSO₃H, n is 0, 1, 2 or 3, A is a spacer selected from the group consisting of CH₂, CH(OH), CH(CH₃), CH₂CH₂, CH(CH₃)CH₂, CH₂CH(CH₃), CH(OH)CH₂, CH₂CH(OH), (Z)CH=CH, (E)CH=CH, C(=O)NHCH₂, preferably CH₂CH₂, (E)CH=CH, m is 0, 1, 2 or 3, B is selected from the group consisting of OH, C(=O)H, C(=O)OH, OCH₃, C(=O)OCH₃, C(=O)OCH₂CH₃, OSO₃H, OSO₃CH₃, OSO₃CH₂CH₃.

Particularly preferred [R]n substituents comprise OH, OCH₃, OSO₃H, n is preferably 1, 2 or 3, m is preferably 0, A is preferably selected from CH₂CH₂, (E)CH=CH, B is preferably selected from C(=O)OH, SO₃H. Many such compounds exhibit antioxidant and anti-inflammatory activity. While any such compounds can be comprised within the claimed composition in accordance with the philosophy of the present invention, some preferred compounds are: 3,4,5-trihydroxybenzoic acid, 2-hydroxybenzoic acid, ethyl 3,4,5-trihydroxybenzoate, 3-(3-hydroxy-4-methoxyphenyl)propanoic acid, 3-hydroxy-3-(3-hydroxyphenyl)propanoic acid, 3-(4-hydroxy-3-methoxyphenyl)propanoic acid, 3-hydroxy-4-methoxy-5-(sulfoxy)benzoic acid, benzoic acid, (E)-3-(3,5-dimethoxy-4-hydroxyphenyl)-2-propenoic acid, 3-hydroxybenzoic acid, 3,5-dihydroxy-4-methoxybenzoic acid, 3-(3,4-dihydroxyphenyl)propanoic acid, 3,4-dihydroxy-5-methoxybenzoic acid, 3,4-dihydroxybenzoic acid, 3-methoxy-4-(sulfoxy)benzoic acid, (E)-3-phenylprop-2-enoic acid or salts thereof, most preferably 3,4,5-trihydroxybenzoic acid, 2-hydroxybenzoic acid, 3,5-dihydroxyphenyl hydrogen sulfate (phloroglucinol-O-sulfate), 2,6-dihydroxyphenyl hydrogen sulfate, 3-hydroxyphenyl hydrogen sulphate (resorcinol-O-sulfate) and salts thereof.

In another embodiment, the composition of the present invention is useful for treating neurodegenerative disorders by inhibiting the activation of the production of pro-inflammatory cytokines, said pro-inflammatory cytokines comprising G-CSF, TNF-α, TGFβ1, IL-6, IL-10, IL-1β, IL-12, IL-18, IL23, CCL17, CCL22 and CXCL1, preferably TNF-α, IL-6, IL-1β.

Still in another embodiment, the composition of the present invention is useful for treating neurodegenerative disorders by promoting the inactivation of pro-inflammatory transcription factors through regulatory protein IkB-α, said pro-inflammatory transcription factors comprising NF-kB.

In yet another embodiment, the composition of the present invention is useful for treating neurodegenerative disorders by reducing TLR4 receptor presence in outer cell membrane. Neurodegenerative disorders can comprise, but is not limited to, Alzheimer's disease, Parkinson's diseases, amyotrophic lateral sclerosis, muscular dystrophy, multiple sclerosis, brain cancer, epilepsy, frontotemporal dementia.

In another aspect, the composition of the present invention can be used to prepare nutraceutical supplement or food product for preventing neurodegenerative disorders through oral administration, said formulations being liquid, powdered, dried, lyophilised.

Liposomal or proteinoid encapsulation may be used to formulate the composition. Liposomal encapsulation may be used and the liposomes may be derivatized with various polymers. In general, the formulation will include inert ingredients for protecting the composition in the stomach environment, and release of the biologically active material in the intestine. Nutraceutical compositions of the present invention can optionally include other botanic and/or herbal extracts to maintain, restore and improve health benefits.

Still in another aspect, the composition of the present invention can be used to prepare pharmaceutical formulations for treating neurodegenerative disorders through oral administration, said formulations being solid, such as for example tablets, pills, hard capsules, and liquid, such us for example beverages, syrups, vials, drops and other dosage forms in accordance with the Art.

Pharmaceutical formulations can comprise pharmaceutically acceptable vehicles and/or excipients and/or carriers. Excipients can comprise, for example: anti-adherents, antioxidants, binders, coatings, compression aids, disintegrants, dyes (colours), emollients, emulsifiers, fillers (diluents), film formers or coatings, flavours, fragrances, glidants (flow enhancers), lubricants, preservatives, printing inks, sorbents, suspension or dispersing agents, sweeteners, and waters of hydration. Exemplary excipients comprise, but are not limited to: butylated hydroxytoluene (BHT), calcium carbonate, calcium phosphate (dibasic), calcium stearate, calcium sulfate, croscarmellose, crosslinked polyvinyl pyrrolidone, citric acid, crospovidone, cysteine, ethyl cellulose, gelatine, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

Those skilled in the Art will appreciate the intrinsic possibility to use the composition of the present invention in therapeutic combination with other anti-inflammatory agents. In other aspects, the anti-inflammatory agents can include pharmaceutical non steroidal and steroidal ones. Representative examples of non-steroidal anti-inflammatory agents comprise, without limitation, oxicams, such as piroxicam, isoxicam, tenoxicam, sudoxicam; salicylates, such as aspirin, disalcid, benorylate, trilisate, safapryn, solprin, diflunisal, and fendosal; acetic acid derivatives, such as diclofenac, fenclofenac, indomethacin, sulindac, tolmetin, isoxepac, furofenac, tiopinac, zidometacin, acematacin, fentiazac, zomepirac, clindanac, oxepinac, felbinac, and ketorolac; fenamates, such as mefenamic, meclofenamic, flufenamic, niflumic, and tolfenamic acids; propionic acid derivatives, such as ibuprofen, naproxen, benoxaprofen, flurbiprofen, ketoprofen, fenoprofen, fenbufen, indopropfen, pirprofen, carprofen, oxaprozin, pranoprofen, miroprofen, tioxaprofen, suprofen, alminoprofen, and tiaprofenic; pyrazoles, such as phenylbutazone, oxyphenbutazone, feprazone, azapropazone, and trimethazone. Mixtures of these non-steroidal anti-inflammatory agents may also be employed.

Representative examples of steroidal anti-inflammatory agents comprise, without limitation, corticosteroids such as hydrocortisone, hydroxyl-triamcinolone, alpha-methyl dexamethasone, dexamethasone-phosphate, beclomethasone dipropionates, clobetasol valerate, desonide, desoxymethasone, desoxycorticosterone acetate, dexamethasone, dichlorisone, diflorasone diacetate, diflucortolone valerate, fluadrenolone, fluclorolone acetonide, fludrocortisone, flumethasone pivalate, fluosinolone acetonide, fluocinonide, flucortine butylesters, fluocortolone, fluprednidene (fluprednylidene) acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenolone, fludrocortisone, diflurosone diacetate, fluradrenolone acetonide, medrysone, amcinafel, amcinafide, betamethasone and the balance of its esters, chloroprednisone, chlorprednisone acetate, clocortelone, clescinolone, dichlorisone, diflurprednate, flucloronide, flunisolide, fluoromethalone, fluperolone, fluprednisolone, hydrocortisone valerate, hydrocortisone cyclopentylpropionate, hydrocortamate, meprednisone, paramethasone, prednisolone, prednisone, beclomethasone dipropionate, triamcinolone, and mixtures thereof.

Embodiments of the present invention are illustrated by the following examples, which are not to be construed in any way as imposing limitations upon the scope thereof. On the contrary, it is to be clearly understood that resort may be had to various other embodiments, modifications, and equivalents thereof, which, after reading the description herein, may suggest themselves to those skilled in the Art without departing from the spirit of the invention. During the studies described in the following examples, conventional procedures of medicinal chemistry and pharmacology were followed, unless otherwise stated. Some of the procedures are described below for illustrative purpose about the efficacy of the composition of the present invention.

### EXAMPLES

### Materials and methods:

Solvent and chemicals were purchased and used substantially pure without further purification. Polyphenol metabolites were purchased and used substantially pure without further purification and/or synthetized according to well established chemical routes known to those skilled in the Art. Synthetized compounds were obtained as sodium salts and were firstly dissolved in DMSO prior to dilution to final concentration in specific cell media.

The "N9" murine microglia cell line was cultured in EMEM (Eagle Minimum Essential Media) supplemented with 10% FBS, 200 nM L-glutamine, 1% NEAA and maintained at 37 °C, 5% CO₂. Experiments were performed on 24-well plates. Compounds were incubated in cells for the indicated amount of time (2, 4 or 6 hours) before the addition of the indicated inflammatory stimuli (LPS or TNFα:IFNγ).

The LUHMES cells (human dopaminergic cell line) were cultured in advanced DMEM-F12 (Dulbecco's Minimal Essential Media) at 37°C with 5% CO₂. Experiments were performed in 24 well plates. Compounds were incubated for 24 hours before addition of Parkinson inducing toxin MPP+ was added to cells.

A variety of immunoassay protocols for measuring protein amount or activity are known in the Art. Examples of immunoassay methods comprise, but are not limited to, radioimmunoassay, radio-immunoprecipitation, immunoprecipitation, fluorescent activated cell sorting (FACS), ELISA (enzyme-linked immunosorbent assay), described in literature (Enzyme Immunoassay, E.T. Maggio, ed., CRC Press, Boca Raton, Fla., 1980; Gaastra W., Enzyme-linked immunosorbent assay (ELISA), in Methods of Molecular Biology, Vol. 1, Walker J.M. ed., Humana Press, NJ, 1984).

### EXAMPLE 1: Reduction in LPS-induced production of TNF-α by polyphenol metabolites in microglia cells.

The experiment was conducted *in vitro* using N9 mouse microglia cell line. Polyphenol metabolites were pre-incubated for 2, 4, 6 hours with the cells in the cell media. Afterwards cells were washed and incubated with lipopolysaccharides for 24 hours. At the end of this period cell media was collected and the presence of TNF-α analyzed using ELISA, thus showing the results as substantially depicted in Fig.1.

### EXAMPLE 2: Reduction in LPS-induced production of multiple cytokines by polyphenol metabolites in microglia cells.

The experiment was conducted *in vitro* using N9 mouse microglia cell line. Polyphenol metabolites were pre-incubated for 6 hours in the cell media at the indicated concentration. Afterwards cells were washed and incubated with lipopolysaccharides for 24 hours. At the end of this period cell media was collected and the presence of various cytokines analyzed using flow cytometry (LEGENDplex^{™} Mouse Macrophage/Microglia Panel), thus showing the results as substantially depicted in Fig.2.

### EXAMPLE 3: Reduction in TNF-α and IFNγ -induced production of multiple cytokines by polyphenol metabolites in microglia cells.

The experiment was conducted *in vitro* using N9 mouse microglia cell line. Polyphenol metabolites were pre-incubated for 6 hours in the regular cell media at the indicated concentration. Afterwards cells were washed and incubated with TNF-α 10nM plus IFNγ 1nM for 24 hours. At the end of this period cell media was collected and the presence of various cytokines analyzed using flow cytometry (LEGENDplex^{™} Mouse Macrophage/Microglia Panel), thus showing the results as substantially depicted in Fig.3.

### EXAMPLE 4: Therapeutical model for reduction in LPS-induced production of TNF-α by polyphenol metabolites in microglia cell.

The experiment was conducted *in vitro* using N9 mouse microglia cell line. Cells were incubated with 300ng/mL of lipopolysaccharides. After 2 hours polyphenol metabolites were added to cell medium at concentration as substantially depicted in Fig.4. Cell media was collected at 2, 4 and 6 hours and analyzed for the presence of TNF-α by ELISA.

### EXAMPLE 5: Effect of polyphenol metabolites on cell viability and ATP levels.

The experiment was conducted *in vitro* using "LUHMES", human dopaminergic cell line at 37°C with 5% CO₂. Polyphenol metabolites were incubated for 24 hours at 5 µM. After this period cells were washed and incubated (or not) with MPP+ (1-phenyl-4-methylpyridinium ion) for 24 hours in order to produce the oxidative insult. Afterwards cell medium was replaced with new medium including the cell metabolic dyes: resazurin for 2 hours or luciferase for 30 min. Colour development was measured by fluorescence and luminescence respectively, in a Synergy (Biotek) plate reader, thus showing the results as substantially depicted in Fig.5.

### EXAMPLE 6: Reduction of TLR4 in the outer cell membrane.

The experiment was conducted *in vitro* using N9 mouse microglia cell line. Polyphenol metabolites were pre-incubated for 6 hours in the regular cell media. Afterwards cells were washed and incubated with TLR4-PE antibody for 30 minutes. Afterwards cells were washed and the amount of TLR4 in the cell membrane measured by flow cytometry. Results are shown as percentage of TLR4 relative to control as substantially depicted in Fig.6.

## Claims

1. A polyphenol metabolites-based composition comprising at least one therapeutically effective amount compound of Formula (I) 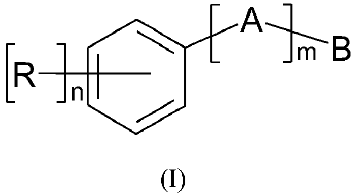 or salts thereof, wherein:
[R]n is one radical R if n is 1 or represents n radicals R attached to different position of the basic ring if n is more than 1, and wherein each R is independently selected from the group consisting of H, OH, OCH₃, OCH₂CH₃, OSO₃H, OSO₃CH₃, OSO₃CH₂CH₃, F, Cl, Br, I, n is 0, 1, 2 or 3, A is a spacer selected from the group consisting of CH₂, CH(OH), CH(CH₃), CH₂CH₂, CH(CH₃)CH₂, CH₂CH(CH₃), CH(OH)CH₂, CH₂CH(OH), (Z)CH=CH, (E)CH=CH, C(=O)NHCH₂, m is 0, 1, 2 or 3, B is selected from the group consisting of OH, C(=O)H, C(=O)OH, OCH₃, C(=O)OCH₃, C(=O)OCH₂CH₃, OSO₃H, OSO₃CH₃, OSO₃CH₂CH₃, said composition **characterised in that** it inhibits the activation of the production of inflammatory cytokines, further **characterised in that** it inhibits the activity of inflammatory transcription factors, in brain immune innate cells.

2. The composition according to claim 1, wherein [R]n is OH, OCH₃, OSO₃H, n is 1, 2 or 3, A is CH₂CH₂, (E)CH=CH, m is 0, B is C(=O)OH, SO₃H.

3. The composition according to claim 1, said inflammatory cytokines comprising G-CSF, TNF-α, TGFβ1, IL-6, IL-10, IL-1β, IL-12, IL-18, IL23, CCL17, CCL22 and CXCL1.

4. The composition according to claim 3, wherein said inflammatory cytokines are TNF-α, IL-6, IL-1β.

5. The composition according to claim 1, wherein said brain immune innate cells are microglia cells.

6. The composition according to claim 1, said inflammatory transcription factors comprising NF-κB.

7. The composition according to claims 1-6, wherein said polyphenol metabolites are selected from the group consisting of 3-hydroxyphenyl hydrogen sulfate, 3,5-dihydroxyphenyl hydrogen sulfate, 3,4,5-trihydroxybenzoic acid, 2-hydroxybenzoic acid, ethyl 3,4,5-trihydroxybenzoate, 3-(3-hydroxy-4-methoxyphenyl)propanoic acid, 3-hydroxy-3-(3-hydroxyphenyl)propanoic acid, 3-(4-hydroxy-3-methoxyphenyl)propanoic acid, 3-hydroxy-4-methoxy-5-(sulfoxy)benzoic acid, benzoic acid, (E)-3-(3,5-dimethoxy-4-hydroxyphenyl)-2-propenoic acid, 3-hydroxybenzoic acid, 3,5-dihydroxy-4-methoxybenzoic acid, 3-(3,4-dihydroxyphenyl)propanoic acid, 3,4-dihydroxy-5-methoxybenzoic acid, 3,4-dihydroxybenzoic acid, 3-methoxy-4-(sulfoxy)benzoic acid, (E)-3-phenylprop-2-enoic acid or salts thereof.

8. The composition according to claim 7, wherein said polyphenol metabolites are 3-hydroxyphenyl hydrogen sulfate, 3,5-dihydroxyphenyl hydrogen sulfate,

9. The composition according to claim 8, **characterized in that** it reduces TLR4 presence in outer cell membrane.

10. A composition according to anyone of the preceding claims, **characterised in that** said composition is a pharmaceutical composition or nutraceutical supplement or food product.

11. The composition according to claim 10, **characterised in that** said composition is in liquid, powdered, dried, lyophilised form.

12. The composition according to claim 10, **characterized in that** it comprises at least one polyphenol metabolite or pharmaceutically acceptable salts thereof as active ingredient and at least one pharmaceutically acceptable vehicle and/or excipient and/or carrier.

13. The composition according to claim 12, comprising at lest another anti-inflammatory agent as active ingredient.

14. The composition according to claim 10, **characterised in that** said composition is in solid form comprising tablets, pills, hard capsules, powders, granules, and in liquid form comprising beverages, syrups, vials, drops.

15. Use of the composition of claim 10 for the manufacture of a medicament for the prevention and/or treatment of neurodegenerative diseases.
